(19) Europäisches Patentamt
European Patent Office
Office européen des brevets

(11) Publication number: **0 165 032**
**A2**

(12) **EUROPEAN PATENT APPLICATION**

(21) Application number: 85304083.0

(22) Date of filing: 10.06.85

(51) Int. Cl.[4]: C 07 D 401/08
A 61 K 31/505

(30) Priority: 15.06.84 GB 8415386

(43) Date of publication of application: 18.12.85 Bulletin 85/51

(84) Designated Contracting States: AT BE CH DE FR GB IT LI LU NL SE

(71) Applicant: Pfizer Limited
Ramsgate Road
Sandwich Kent CT13 9NJ(GB)

(84) Designated Contracting States: GB

(71) Applicant: Pfizer Corporation
Calle 15 1/2 Avenida Santa Isabel
Colon(PA)

(84) Designated Contracting States: BE CH DE FR IT LI LU NL SE AT

(72) Inventor: Alker, David, Dr.
21, Whitewood Road
Eastry, Nr. Deal Kent(GB)

(72) Inventor: Campbell, Simon Fraser, Dr.
'Grey Friars' Upper Street
Kingsdown Deal Kent(GB)

(72) Inventor: Cross, Peter Edward, Dr.
21, Cherry Avenue
Canterbury Kent(GB)

(74) Representative: Moore, James William
Pfizer Limited Ramsgate Road
Sandwich Kent CT13 9NJ(GB)

(54) **Dihydropyridine anti-ischaemic and antihypertensive agents.**

(57) 1, 4-Dihydropyridines of the formula:–

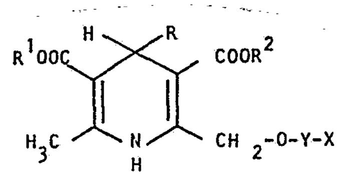

wherein R is aryl or heteroaryl; $R_1$ and $R_2$ are each $C_1$–$C_4$ alkyl or 2-methoxyethyl; X is a 5 or 6 memebred nitrogen-containing aromatic heterocyclic ring which is substituted with one or more $C_1$–$C_4$ alkoxy groups and which may optionally be additionally substituted with one or more $C_1$–$C_4$ alkyl, aryl, hydroxy, oxo, CN, $N(R^4)_2$, $(CH_2)_mCON(R^4)_2$ or $(CH_2)_mCO_2R^5$ groups, wherein m is 0 or 1, each $R^4$ is independently H or $C_1$–$C_4$ alkyl or the two groups $R_4$ are taken together with the nitrogen atom to which they are attached to form a pyrrolidinyl, piperidino, morpholino, piperazinyl or N–($C_1$–$C_4$ alkyl)–piperazinyl group and $R^5$ is H or $C_1$–$C_4$ alkyl; and Y is $-(CH_2)_n-$, $-CH_2CH(CH_3)-$ or $-CH_2C(CH_3)_2-$ wherein n is 1 to 3; are calcium channel blockers useful as anti-ischaemic and antihypertensive agents.

EP 0 165 032 A2

This invention relates to certain dihydropyridines, specifically to certain 1,4-dihydropyridines having a substituted heterocyclic ring in a side chain attached to the 2-position, which have utility as anti-ischaemic and antihypertensive agents, and to pharmaceutical preparations containing such compounds.

The compounds of the invention reduce the movement of calcium into the cell and they are thus able to delay or prevent the cardiac contracture which is believed to be caused by an accumulation of intracellular calcium under ischaemic conditions. Excessive calcium influx during ischaemia can have a number of additional adverse effects which would further compromise the ischaemic myocardium. These include less efficient use of oxygen for ATP production, activation of mitochondrial fatty acid oxidation and possibly, promotion of cell necrosis. Thus the compounds are useful in the treatment or prevention of a variety of cardiac conditions, such as angina pectoris, cardiac arrhythmias, heart attacks and cardiac hypertrophy. The compounds also have vasodilator activity since they can inhibit calcium influx in cells of vascular tissue and they are thus also useful as antihypertensive agents and for the treatment of coronary vasospasm.

According to the specification of our European patent application publication no. 60674 there are described and claimed a number of dihydropyridine anti-ischaemic and antihypertensive agents wherein the 2-position of the dihydropyridine ring is substituted with certain N,N-disubstituted aminoalkoxymethyl groups. Our European patent application publication no. 100189

describes and claims a related series of compounds wherein the 2-position is substituted with an aromatic heterocyclylalkoxymethyl group. We have now discovered a further series of dihydropyridine compounds having valuable therapeutic properties wherein the 2-position substituent bears an alkoxy-substituted-heterocyclylalkoxymethyl group.

Thus according to the invention, there are provided novel 1,4-dihydropyridine derivatives of the formula:-

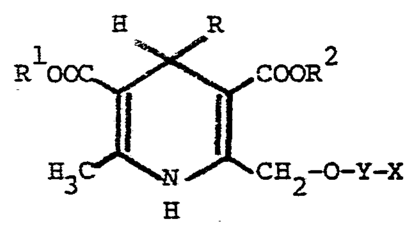

--- (I)

and their pharmaceutically acceptable salts;

wherein R is aryl or heteroaryl;

$R^1$ and $R^2$ are each independently $C_1$-$C_4$ alkyl or 2-methoxyethyl;

X is a 5 or 6 membered nitrogen-containing aromatic heterocyclic ring which is substituted with one or more $OR^3$ groups and which may optionally be additionally substituted with one or more $C_1$-$C_4$ alkyl, aryl, hydroxy, oxo, CN, $N(R^4)_2$, $(CH_2)_mCON(R^4)_2$ or $(CH_2)_mCO_2R^5$ groups;

$R^3$ is $C_1$-$C_4$ alkyl;

each $R^4$ is independently H or $C_1$-$C_4$ alkyl or the two groups $R^4$ are taken together with the nitrogen atom to which they are attached to form a pyrrolidinyl, piperidino, morpholino, piperazinyl or N -($C_1$-$C_4$ alkyl)-piperazinyl group;

$R^5$ is H or $C_1$-$C_4$ alkyl;

Y is $-(CH_2)_n-$, $-CH_2CH(CH_3)-$ or $-CH_2C(CH_3)_2-$;

m is 0 or 1;

and n is 1 to 3 when X is linked to Y by a ring carbon atom, or 2 or 3 when X is linked to Y by a ring nitrogen atom.

The pharmaceutically acceptable acid addition salts of the compounds of the formula (I) are those formed from acids which form non-toxic acid addition salts, for example the hydrochloride, hydrobromide, sulphate or bisulphate, phosphate or acid phosphate, acetate, citrate, fumarate, gluconate, lactate, maleate, succinate and tartrate salts. For compounds with an acidic substituent salts may also be formed with bases; examples are the sodium, potassium and ammonium salts.

The term "aryl" as used in this specification includes phenyl and phenyl substituted by one or two substituents each independently selected from nitro, halo, $C_1$-$C_4$ alkyl, $C_1$-$C_4$ alkoxy, hydroxy, trifluoromethyl and cyano. It also includes 1- and 2-naphthyl.

The term "heteroaryl" as used in this specification for R means an aromatic heterocyclic group which may optionally be substituted and includes, for example, benzofuranyl; benzothienyl; pyridyl optionally substituted by methyl, methylthio, cyano or halo; quinolyl; benzoxazolyl; benzothiazolyl; furyl; pyrimidinyl; thiazolyl; 2,1,3-benzoxadiazol-4-yl; 2,1,3-benzothiadiazol-4-yl; and thienyl optionally monosubstituted by halo or $C_1$-$C_4$ alkyl.

The 5 or 6 membered nitrogen-containing aromatic heterocyclic ring X may contain from one to three nitrogen atoms in the ring and may be linked to the group Y either by a ring carbon or a ring nitrogen atom, with the proviso that, if X is linked by a ring nitrogen atom, Y must contain at least two carbon atoms (i.e. n is 2 or 3). Examples of suitable heterocyclic rings include pyrimidinyl, pyrazolinyl, imidazolyl and triazolyl.

The substituent $OR^3$ is preferably methoxy. In addition the $OR^3$ group, the heterocyclic ring X may contain further substituents. Preferred substituent groups are amino, $C_1$-$C_4$ alkyl, particularly methyl, and hydroxy or oxo groups. As will be understood by those skilled in the art the hydroxy- or oxo-substituents, if present, can be subject to keto-enol tautomerism and the group may be present either as a free hydroxyl group when in the tautomeric enol form, or as an oxo group when in the keto form.

Thus particular examples of X include 4-methoxy-2-methylpyrimidin-6-yl; 4-isopropoxy-2-methylpyrimidin-6-yl; 2-amino-4-methoxypyrimidin-6-yl, 2-methoxy-4-pyrimidon-6-yl, and 2,4-dimethoxypyrimidin-6-yl.

As used herein the term "halo" means fluoro, chloro, bromo or iodo. Alkyl and alkoxy groups having 3 or more carbon atoms can be straight or branched chain. Compounds containing asymmetric centres will exist as one or more pairs of enantiomers and the invention includes the separated d- and l- optically active isomers as well as mixtures thereof.

Preferred values for R are 2-chlorophenyl and 2,3-dichlorophenyl. $R^1$ and $R^2$ are preferably $CH_3$ or $C_2H_5$; the case where $R^1$ is $CH_3$ and $R^2$ is $C_2H_5$ being especially preferred. Y is preferably $-(CH_2)_2-$ when X is linked to Y by a ring nitrogen atom, or $-CH_2-$ when X is linked to Y by a ring carbon atom. Thus particular examples of compounds of the invention include the following:

6-{[4-(2,3-Dichlorophenyl)-3-ethoxycarbonyl-5-methoxycarbonyl-6-methyl-1,4-dihydropyrid-2-yl]methoxymethyl}-4-methoxy-2-methylpyrimidine;

6-{[4-(2,3-Dichlorophenyl)-3-ethoxycarbonyl-5-methoxycarbonyl-6-methyl-1,4-dihydropyrid-2-yl]methoxymethyl}-4-isopropoxy-2- methylpyrimidine;

2-Amino-6-{[4-(2,3-dichlorophenyl)-3-ethoxycarbonyl-5-methoxycarbonyl-6-methyl-1,4-dihydropyrid-2-yl]methoxymethyl}-4-methoxypyrimidine;

6-{[3,5-Bismethoxycarbonyl-4-(2,3-dichlorophenyl)-6-methyl-1,4-dihydropyrid-2-yl]methoxymethyl}-2,4-dimethoxypyrimidine and

6-{[4-(2,3-Dichlorophenyl)-3-ethoxycarbonyl-5-methoxycarbonyl-6-methyl-1,4-dihydropyrid-2-yl]methoxymethyl}-2-methoxy-4-pyrimidone.

The compounds of the invention can be prepared by a number of different processes according to the invention.

In one process the compounds of formula I can be prepared from the corresponding compound of the formula:

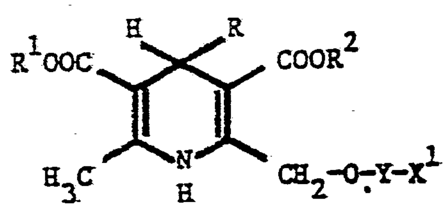

--- (II)

wherein R, $R^1$, $R^2$ and Y are as previously defined and $X^1$ is a 5 or 6 membered nitrogen containing heterocyclic group as defined for X except that the ring does not contain an $OR^3$ substituent group but instead is substituted by halogen, especially chlorine, or $S(C_1-C_4)$alkyl; by reacting with an alkoxide of the formula:

$$R^3-O^{\ominus} \qquad \text{--- (III)}$$

where $R^3$ is as previously defined, formed by reacting an alcohol of the formula $R^3$-OH with a strong base, for example sodium hydride.

The reaction is generally performed by preparing the anion of the alcohol of formula (III) and then adding the compound of formula II in more or less equimolar quantities. For the simple lower alkanols of formula (III) the reaction can be performed using excess alcohol as solvent, and a period of several days at room temperature may be required before the reaction is substantially complete. The desired product is isolated and purified using conventional procedures.

In an alternative procedure for preparing the compounds of formula I wherein $R^3$ is methyl, the corresponding compound of formula II wherein $X^1$ has a hydroxy substituent instead of $OR^3$ is reacted with trimethyloxonium tetrafluoroborate. The reaction is performed in a conventional manner by adding the methylating agent to a cooled solution of the hydroxy-substituted compound in an organic solvent, for example dichloromethane. The reaction is generally complete after several hours at room temperature and the product is isolated and purified using conventional procedures.

The starting materials of formula II wherein $X^1$ is substituted with hydroxy groups are described in our co-pending British Patent application number 8319886. The starting compounds of formula II wherein $X^1$ is substituted with halo groups are prepared from the corresponding hydroxy-substituted compounds; for example chloro-substituted compounds can be prepared from the hydroxy-substituted starting materials by reaction with phosphorus oxychloride.

In a further process, compounds of the formula I wherein X is a 2-methoxy-4-pyrimidon-6-yl group are prepared from a compound of the formula:

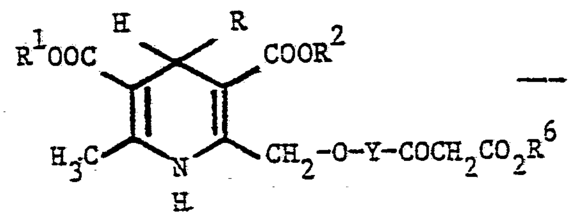

— (IV)

wherein R, $R^1$, $R^2$ and Y are as previously defined and $R^6$ is $C_1$-$C_4$ alkyl; by reacting with O-methylisourea hydrogen sulphate.

The reaction is performed with the reactants dissolved in an organic solvent e.g. ethanol, in the presence of a base e.g. 1,5-diazabicyclo[4.3.0]non-5-ene. A period of several days at room temperature may be required and the product is then isolated and purified by conventional procedures.

The starting materials of formula IV are described in our British patent application no. 8319886.

The ability of the compounds to inhibit the movement of calcium into the cell is shown by their effectiveness in reducing the contraction of vascular tissue in vitro which is the consequence of calcium influx caused by a high extracellular concentration of potassium ions. The test is performed by mounting spirally cut strips of rat aorta with one end fixed and the other attached to a force transducer. The tissue is immersed in a bath of physiological saline solution containing calcium ions at a concentration of 2.5 millimolar and potassium ions at a concentration of 5.9 millimolar. Potassium chloride solution is added to the bath with a pipette to give a final potassium ion concentration of 45 millimolar. The change in tension caused by the resulting contraction of the tissue is noted. The bath is drained and refilled with fresh saline solution containing the particular compound under test. After 45 minutes, the procedure is repeated and the contraction again noted. The concentration of compound required to reduce the response by 50% is determined.

The antihypertensive activity of the compounds is evaluated after oral administration by measuring the fall in blood pressure in spontaneously hypertensive rats or renally hypertensive dogs.

For administration to man in the curative or prophylactic treatment of cardiac conditions and hypertension, oral dosages of the compounds will generally be in the range of from 20-200 mg daily for an average adult patient (70 kg). Thus for a typical adult patient, individual tablets or capsules contain from 5 to 20 mg of active compound, in a suitable pharmaceutically acceptable

vehicle or carrier. Dosages for intravenous administration would typically be within the range 1 to 10 mg per single dose as required. In practice the physician will determine the actual dosage which will be most suitable for an individual patient and it will vary with the age, weight and response of the particular patient. The above dosages are exemplary of the average case but there can, of course, be individual instances where higher or lower dosage ranges are merited, and such are within the scope of this invention.

For human use, the compounds of the formula (I) can be administered alone, but will generally be administered in admixture with a pharmaceutical carrier selected with regard to the intended route of administration and standard pharmaceutical practice. For example, they may be administered orally in the form of tablets containing such excipients as starch or lactose, or in capsules or ovules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other substances, for example, enough salts or glucose to make the solution isotonic with blood.

Thus in a further aspect the invention provides a pharmaceutical composition comprising a compound of the formula (I), or a pharmaceutically acceptable salt thereof, together with a pharmaceutically acceptable diluent or carrier.

The invention also includes a compound of the formula (I), or a pharmaceutically acceptable salt thereof, for use in medicine, in particular in the treatment of ischaemic heart disease, angina, or hypertension in a human being.

The preparation of the compounds of the invention is illustrated by the following Examples.

EXAMPLE 1

6-{[4-(2,3-Dichlorophenyl)-3-ethoxycarbonyl-5-methoxycarbonyl-6-methyl-1,4-dihydropyrid-2-yl]methoxymethyl}-4-methoxy-2-methylpyrimidine

Sodium hydride (50 mg; 60% dispersion in oil) was added to methanol (20 ml). 4-Chloro-6-{[4-(2,3-dichlorophenyl)-3-ethoxycarbonyl-5-methoxycarbonyl-6-methyl-1,4-dihydropyrid-2-yl]methoxymethyl}-2-methylpyrimidine (0.33 g) was then added to the resulting solution and the mixture stirred at room temperature for 3 days and then evaporated. The residue was partitioned between ethyl acetate and water and the organic layer washed once with brine, dried over magnesium sulphate and evaporated. The residue was crystallised from diethyl ether to give the title compound (0.20 g), m.p. 145-149°C. Found: C,55.70; H,5.00; N,7.92. $C_{25}H_{27}Cl_2N_3O_6$ requires: C,55.98; H,5.07; N,7.83%.

EXAMPLE 2

6-{[4-(2,3-Dichlorophenyl)-3-ethoxycarbonyl-5-methoxycarbonyl-6-methyl-1,4-dihydropyrid-2-yl]methoxymethyl}-4-isopropoxy-2-methylpyrimidine was prepared by the method described in Example 1 but using isopropanol instead of methanol. The product had m.p. 156-160°C. Found: C,56.98; H,5.52; N,7.39. $C_{27}H_{31}Cl_2N_3O_6$ requires: C,57.45; H,5.54; N,7.44%.

EXAMPLE 3

2-Amino-6-{[4-(2,3-dichlorophenyl)-3-ethoxycarbonyl-5-methoxycarbonyl-6-methyl-1,4-dihydropyrid-2-yl]methoxymethyl}-4-methoxypyrimidine

Trimethyloxonium tetrafluoroborate (0.85 g) was added to a stirred suspension of 2-amino-6-{[4-(2,3-dichlorophenyl)-3-ethoxycarbonyl-5-methoxycarbonyl-6-methyl-1,4-dihydropyrid-2-yl]methoxymethyl}-4-pyrimidone (1.00 g) in dichloromethane (100 ml) with cooling in an ice/acetone bath. The mixture was then stirred at room temperature for 24 hours, washed twice with 5% aqueous sodium carbonate solution, dried over magnesium sulphate and evaporated. The residue was purified by chromatography on silica (5 g) using hexane plus 60-90% by volume of dichloromethane followed by dichloromethane plus 0-1% methanol by volume as eluant. Appropriate fractions were combined and evaporated and the residue triturated with diethyl ether. The resulting solid was collected, washed with diethyl ether and dried to give the title compound (80 mg), m.p. 160-162°C. Found: C,53.81; H,5.07; N,10.32. $C_{24}H_{26}Cl_2N_4O_6$ requires: C,53.64; H,4.89; N,10.43%.

EXAMPLE 4

6-{[4-(2,3-Dichlorophenyl)-3-ethoxycarbonyl-5-methoxycarbonyl-6-methyl-1,4-dihydropyrid-2-yl]methoxymethyl}-2-methoxy-4-pyrimidone

A mixture of ethyl 4-{[4-(2,3-dichlorophenyl)-3-ethoxycarbonyl-5-methoxycarbonyl-6-methyl-1,4-dihydropyrid-2- yl]methoxy}-acetoacetate (0.53 g), O-methylisourea hydrogen

sulphate (0.20 g) and 1,5-diazabicyclo[4,3.0]non-5-ene (0.25 g) in ethanol was stirred at room temperature for 7 days and then evaporated. The residue was partitioned between water and chloroform and the organic layer washed with 2M hydrochloric acid and water, dried over sodium sulphate and evaporated. The residue was triturated with diethyl ether and the resulting solid collected, washed with diethyl ether and dried to give the title compound (34 mg), m.p. 199-200°C decomp. Found: C,54.15; H,4.79; 7.50. $C_{24}H_{25}Cl_2N_3O_7$ requires: C,53.54; H,4.68; N,7.80%.

## EXAMPLE 5

6-{[3,5-Bismethoxycarbonyl-4-(2,3-dichlorophenyl)-6-methyl-1,4-dihydropyrid-2-yl]methoxymethyl}-2,4-dimethoxypyrimidine

A solution of 4-chloro-6-{[4-(2,3-dichlorophenyl)-3-ethoxycarbonyl-5-methoxycarbonyl-6-methyl-1,4-dihydropyrid-2-yl]methoxymethyl}-2-methylthiopyrimidine (0.28 g) in methanol (20 ml) containing sodium hydride (0.15 g as an 80% dispersion in oil) was heated under reflux for 4 hours and then evaporated. The residue was partitioned between ethyl acetate and water and the organic layer washed with water, dried over sodium sulphate and evaporated. The residue was purified by chromatography on silica (5 g) using toluene plus 0-25% by volume of ethyl acetate as eluant. Appropriate fractions were combined and evaporated to give the title compound (78 mg), m.p. 144-146°C. Found: C,53.39; H,4.78; N,7.68. $C_{24}H_{25}Cl_2N_3O_7$ requires: C,53.53; H,4.67; N,7.81%.

## Preparation 1

4-Chloro-6-{[4-(2,3-dichlorophenyl)-3-ethoxycarbonyl-5-methoxycarbonyl-6-methyl-1,4-dihydropyrid-2-yl]methoxymethyl}-2-methylpyrimidine

A mixture of 6-{[4-(2,3-dichlorophenyl)-3-ethoxycarbonyl-5-methoxycarbonyl-6-methyl-1,4-dihydropyrid-2-yl]methoxymethyl}-2-methyl-4-pyrimidone (3.0 g), phosphorus oxychloride (15 ml) and triethylamine (1 ml) in chloroform (45 ml) was stirred for 16 hours at room temperature and evaporated. The residue was partitioned between ethyl acetate and water and the organic layer washed twice with saturated aqueous sodium hydrogen carbonate solution, dried over magnesium sulphate and evaporated to give the title compound (2.5 g), m.p. 144-149°C.

## Preparation 2

Ethyl 4-{[4-(2,3-dichlorophenyl)-3-ethoxycarbonyl-5-methoxycarbonyl-6-methyl-1,4-dihydropyrid-2-yl]methoxy}acetoacetate

A solution of carbonyl diimidazole (5.20 g) and 2-{[4-(2,3-dichlorophenyl)-3-ethoxycarbonyl-5-methoxycarbonyl-6-methyl-1,4-dihydropyrid-2-yl]methoxy}acetic acid (14.00 g) in dichloromethane (200 ml) was stirred at room temperature under nitrogen for 2 hours and then added to a solution of pyridine (2.40 g) and 2,2-dimethyl-1,3-dioxane-4,6-dione (4.56 g) in dichloromethane (200 ml) over 8 minutes. The mixture was stirred at room temperature for 2.5 hours, washed successively with water, ice-cold 2.5 M hydrochloric acid and saturated brine, dried over anhydrous magnesium sulphate and evaporated. The residue was

dissolved in ethanol (200 ml) and the solution heated under reflux for 2.75 hours and evaporated. The residue was triturated with diethyl ether and the resulting solid collected, washed with diethyl ether and dried to give the title compound (9.0 g), m.p. 99-102°C.

## Preparation 3

6-{[4-(2,3-Dichlorophenyl)-3-ethoxycarbonyl-5-methoxycarbonyl-6-methyl-1,4-dihydropyrid-2-yl]methoxymethyl}-2-methylthio-4-pyrimidone

A solution of ethyl 4-{[4-(2,3-dichlorophenyl)-3-ethoxycarbonyl-5-methoxycarbonyl-6-methyl-1,4-dihydropyrid-2-yl]methoxy}acetoacetate (1.00 g), S-methylisothiouronium sulphate (0.53 g) and 1,5-diazabicyclo[4.3.0]non-5-ene (0.35 g) in ethanol (30 ml) was stirred at room temperature for 5 days and then evaporated. The residue was partitioned between ethyl acetate and water and the organic layer washed with dilute hydrochloric acid at pH2, dried ($Na_2SO_4$) and evaporated. The residue was crystallised from diethyl ether to give the title compound (0,50 g), m.p. 230-234°C decomp. Found: C,51.88; H,4.77; N,7.24. $C_{24}H_{25}Cl_2N_3O_6S$ requires: C,51.99; H,4.55; N,7.58%.

Preparation 4

4-Chloro-6-{[4-(2,3-dichlorophenyl)-3-ethoxycarbonyl-5-methoxycarbonyl-6-methyl-1,4-dihydropyrid-2-yl]methoxymethyl}-2-methylthiopyrimidine

A solution of 6-{[4-(2,3-dichlorophenyl)-3-ethoxycarbonyl-5-methoxycarbonyl-6-methyl-1,4-dihydropyrid-2-yl]methoxymethyl}-2-methylthio-4-pyrimidone (5.27 g), triethylamine (5 ml) and phosphorus oxychloride (50 ml) in chloroform (150 ml) was stirred at room temperature for 24 hours and then evaporated. The residue was partitioned between ethyl acetate and water and the organic layer washed with water, dried over sodium sulphate and evaporated. The residue was purified by chromatography on silica (50 g) using toluene plus 0-20% by volume of ethyl acetate as eluant. Appropriate fractions were combined and evaporated to give the title compound (2.92 g), m.p. 145-146°C. Found: C,50.45; H,4.30; N,7.43. $C_{24}H_{24}Cl_3N_3O_5S$ requires: C,50.31; H,4.19; N,7.34%.

CLAIMS

1. A compound having the formula:

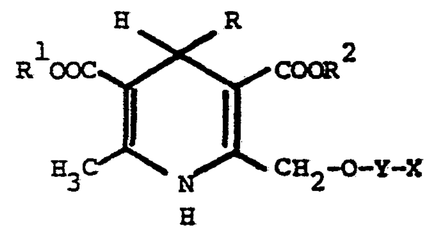

--- (I)

and pharmaceutically acceptable salts thereof;

wherein R is aryl or heteroaryl;

$R^1$ and $R^2$ are each independently $C_1$-$C_4$ alkyl or 2-methoxyethyl;

X is a 5 or 6 membered nitrogen-containing aromatic heterocyclic ring which is substituted with one or more $OR^3$ groups and which may optionally be additionally substituted with one or more $C_1$-$C_4$ alkyl, aryl, hydroxy, oxo, CN, $N(R^4)_2$, $(CH_2)_mCON(R^4)_2$ or $(CH_2)_mCO_2R^5$ groups;

$R^3$ is $C_1$-$C_4$ alkyl;

each $R^4$ is independently H or $C_1$-$C_4$ alkyl or the two groups $R^4$ are taken together with the nitrogen atom to which they are attached to form a pyrrolidinyl, piperidino, morpholino, piperazinyl or N -($C_1$-$C_4$ alkyl)-piperazinyl group.

$R^5$ is H or $C_1$-$C_4$ alkyl;

Y is $-(CH_2)_n-$, $-CH_2CH(CH_3)-$ or $-CH_2C(CH_3)_2-$;

m is 0 or 1;

and n is 1 to 3 when X is linked to Y by a ring carbon atom, or 2 or 3 when X is linked to Y by a ring nitrogen atom.

2. A compound according to claim 1, wherein R is "aryl" and "aryl" is a phenyl or phenyl substituted by one to two substituents each independently selected from nitro, halo, $C_1$-$C_4$ alkyl, $C_1$-$C_4$ alkoxy, hydroxy, trifluoromethyl and cyano, or is 1- or 2-naphthyl; or wherein R is "heteroaryl" and "heteroaryl" is benzofuranyl; benzothienyl; pyridyl optionally substituted by methyl, methylthio, cyano or halo; quinolyl; benzoxazolyl; benzothiazolyl; furyl; pyrimidinyl; thiazolyl; 2,1,3-benzoxadiazol-4-yl; 2,1,3-benzothiadiazol-4-yl or thienyl optionally monosubstituted by halo or $C_1$-$C_4$ alkyl.

3. A compound according to claim 2 wherein R is 2-chlorophenyl or 2,3-dichlorophenyl.

4. A compound according to any one of claims 1 to 3 wherein $R^1$ and $R^2$ are $CH_3$ or $C_2H_5$.

5. A compound according to any one of claims 1 to 4 wherein Y is $CH_2$ and is linked to a ring carbon atom in X.

6. A compound according to any one of claims 1 to 5 wherein X is pyrimidinyl, pyrazolinyl, imidazolyl or triazolyl, which is substituted with one or two $C_1$-$C_4$ alkoxy groups and which is optionally further substituted by amino, $C_1$-$C_4$ alkyl, hydroxy or oxo groups.

7. A compound according to claim 6 where X is 4-methoxy-2-methylpyrimidin-6-yl; 4-isopropoxy-2-methylpyrimidin-6-yl; 2-amino-4-methoxypyrimidin-6-yl, 2-methoxy-4-pyrimidon-6-yl, or 2,4-dimethoxypyrimidin-6-yl.

8. The compound according to claim 1:

6-{[4-(2,3-Dichlorophenyl)-3-ethoxycarbonyl-5-methoxycarbonyl-6-methyl-1,4-dihydropyrid-2-yl]methoxymethyl}-4-methoxy-2-methylpyrimidine;

6-{[4-(2,3-Dichlorophenyl)-3-ethoxycarbonyl-5-methoxycarbonyl-6-methyl-1,4-dihydropyrid-2-yl]methoxymethyl}-4-isopropoxy-2-methylpyrimidine;

2-Amino-6-{[4-(2,3-dichlorophenyl)-3-ethoxycarbonyl-5-methoxycarbonyl-6-methyl-1,4-dihydropyrid-2-yl]methoxymethyl}-4-methoxypyrimidine;

6-{[3,5-Bismethoxycarbonyl-4-(2,3-dichlorophenyl)-6-methyl-1,4-dihydropyrid-2-yl]methoxymethyl}-2,4-dimethoxypyrimidine; or

6-{[4-(2,3-Dichlorophenyl)-3-ethoxycarbonyl-5-methoxycarbonyl-6-methyl-1,4-dihydropyrid-2-yl]methoxymethyl}-2-methoxy-4-pyrimidone.

9. A pharmaceutical composition comprising a compound of the formula (I) as claimed in any one of the preceding claims, or a pharmaceutically acceptable salt thereof, together with a pharmaceutically acceptable diluent or carrier.

10. A compound of the formula (I) as claimed in any one of claims 1 to 8, or a pharmaceutically acceptable salt thereof, for use in medicine including use in the prevention or treatment of cardiac conditions, or use as an antihypertensive, in man.

0165032

CLAIMS for the Contracting State: AT

1. A process for preparing a compound of the formula:

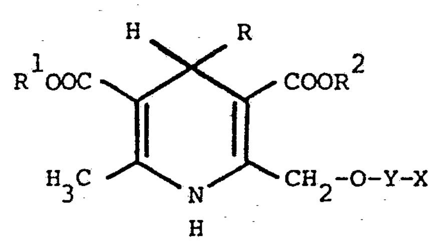

--- (I)

and pharmaceutically acceptable salts thereof;

wherein R is aryl or heteroaryl;

$R^1$ and $R^2$ are each independently $C_1$-$C_4$ alkyl or 2-methoxyethyl;

X is a 5 or 6 membered nitrogen-containing aromatic heterocyclic ring which is substituted with one or more $OR^3$ groups and which may optionally be additionally substituted with one or more $C_1$-$C_4$ alkyl, aryl, hydroxy, oxo, CN, $N(R^4)_2$, $(CH_2)_mCON(R^4)_2$ or $(CH_2)_mCO_2R^5$ groups;

$R^3$ is $C_1$-$C_4$ alkyl;

each $R^4$ is independently H or $C_1$-$C_4$ alkyl or the two groups $R^4$ are taken together with the nitrogen atom to which they are attached to form a pyrrolidinyl, piperidino, morpholino, piperazinyl or N -($C_1$-$C_4$ alkyl)-piperazinyl group;

$R^5$ is H or $C_1$-$C_4$ alkyl;

Y is $-(CH_2)_n-$, $-CH_2CH(CH_3)-$ or $-CH_2C(CH_3)_2-$;

m is 0 or 1;

and n is 1 to 3 when X is linked to Y by a ring carbon atom, or 2 or 3 when X is linked to Y by a ring nitrogen atom, which comprises

(a) reacting a compound of the formula:

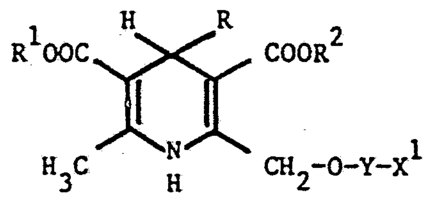

--- (II)

wherein R, $R^1$, $R^2$ and Y are as previously defined and $X^1$ is a 5 or 6 membered nitrogen containing heterocyclic group as defined for X except that the ring does not contain an $OR^3$ substituent group but instead is substituted by halogen, especially chlorine, $S(C_1-C_4)$alkyl or hydroxy; with, in the case where $X^1$ is substituted by halogen or $S(C_1-C_4)$alkyl, an alkoxide of the formula $R^3O^{\ominus}$ where $R^3$ is as previously defined or, in the case where $X^1$ is substituted by OH, with a trialkyloxonium tetrafluoroborate; or

(b) reacting a compound of the formula:

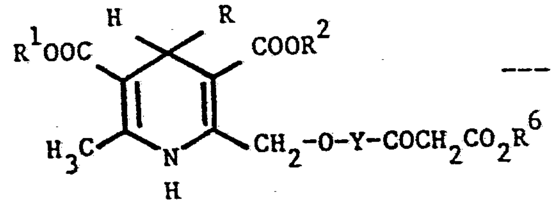

--- (IV)

wherein R, $R^1$, $R^2$, and Y are as previously defined and $R^6$ is $C_1$-$C_4$ alkyl, with O-methylisourea hydrogen sulphate to give compounds wherein X is 2-methoxy-4-pyrimidon-6-yl;

and optionally forming a pharmaceutically acceptable salt of the product.

2. A process according to claim 1 wherein R is 2-chlorophenyl or 2,3-dichlorophenyl.

3. A process according to claim 1 or 2 wherein $R^1$ and $R^2$ are $CH_3$ or $C_2H_5$.

4. A process according to any one of claims 1 to 3 wherein Y is $CH_2$ and is linked to a ring carbon atom in X.

5. A process according to any one of claims 1 to 4 wherein X is pyrimidinyl, pyrazolinyl, imidazolyl or triazolyl, which is substituted with one or two $C_1$-$C_4$ alkoxy groups and which is optionally further substituted by amino, $C_1$-$C_4$ alkyl, hydroxy or oxo groups.

6. A process according to claim 5 wherein X is 4-methoxy-2-methylpyrimidin-6-yl; 4-isopropoxy-2-methylpyrimidin-6-yl; 2-amino-4-methoxypyrimidin-6-yl, 2-methoxy-4-pyrimidon-6-yl or 2,4-dimethoxypyrimidin-6-yl.

7. A process according to claim 1 wherein $X^1$ is substituted by chlorine and the reaction is performed with sodium methoxide in methanol at room temperature.

8. A process according to claim 1 wherein $X^1$ is substituted by hydroxy and the reaction is performed with trimethyloxonium tetrafluoroborate in dichloromethane at 0°C to room temperature.